# EUROPEAN PATENT SPECIFICATION

(11) **EP 3 159 402 B1**
(45) Date of publication and mention of the grant of the patent: **14.04.2021**
(21) Application number: 15810202.0
(22) Date of filing: 18.06.2015
(51) Int. Cl.: C12N 5/07, A61K 35/12, A61K 38/00, A61K 38/22, A61L 27/00, A61P 1/16, A61P 43/00

(54) **TISSUE REGENERATION PROMOTER**
GEWEBEREGENERATIONSPROMOTER
PROMOTEUR DE RÉGÉNÉRATION TISSULAIRE

(30) Priority: 19.06.2014 JP 2014126611
(43) Date of publication of application: 26.04.2017
(73) Proprietor: Nitto Denko Corporation, Ibaraki-shi, Osaka 567-8680 (JP)
(72) Inventor: NIITSU, Yoshiro, Sapporo-shi Hokkaido 064-0823 (JP); YONEDA, Akihiro, Sapporo-shi Hokkaido 060-0005 (JP)
(74) Representative: Hoffmann Eitle
(86) International application number: PCT/JP2015/067567
(87) International publication number: WO 2015/194621

(56) References cited:
- WO-A1-2013/082239
- JP-A- 2001 513 333
- JP-A- 2009 519 042
- JP-A- 2012 527 902
- KISHIDA A ET AL: "RGD-Albumin conjugate: expression of tissue regeneration activity", BIOMATERIALS, ELSEVIER SCIENCE PUBLISHERS BV., BARKING, GB, vol. 13, no. 13, 1 January 1992 (1992-01-01), pages 924-930, XP026025413, ISSN: 0142-9612, DOI: 10.1016/0142-9612(92)90115-5 [retrieved on 1992-01-01]
- ITO A ET AL: "The effect of RGD peptide-conjugated magnetite cationic liposomes on cell growth and cell sheet harvesting", BIOMATERIALS, ELSEVIER SCIENCE PUBLISHERS BV., BARKING, GB, vol. 26, no. 31, 1 November 2005 (2005-11-01), pages 6185-6193, XP027767581, ISSN: 0142-9612 [retrieved on 2005-11-01]
- PARK K-H ET AL: "Phenotype of hepatocyte spheroids in Arg-Gly-Asp (RGD) containing a thermo-reversible extracellular matrix", BIOSCIENCE BIOTECHNOLOGY BIOCHEMIS, JAPAN SOCIETY FOR BIOSCIENCE, BIOTECHNOLOGY, AND AGROCHEMISTRY, TOKYO, JAPAN, vol. 66, no. 7, 1 January 2002 (2002-01-01), pages 1473-1478, XP008090673, ISSN: 0916-8451, DOI: 10.1271/BBB.66.1473
- ORTEGA-VELAZQUEZ R ET AL: "Arg-Gly-Asp-Ser peptide stimulates transforming growth factor-â1 transcription and secretion through integrin activation", THE FASEB JOURNAL, vol. express article, 3 June 2003 (2003-06-03), XP008119316, ISSN: 0892-6638, DOI: 10.1096/FJ.02-0785FJE [retrieved on 2003-06-03]
- JAMES M. KIELY ET AL: "RGD peptides enhance cell proliferation during intestinal adaptation", GASTROENTEROLOGY, vol. 124, no. 4, 1 April 2003 (2003-04-01) , page A597, XP055427296, US ISSN: 0016-5085, DOI: 10.1016/S0016-5085(03)83026-3
- PARK K H: "Improved long-term culture of hepatocytes in a hydrogel containing Arg-Gly-Asp (RGD)", BIOTECHNOLOGY LET, SPRINGER NETHERLANDS, NL, vol. 24, no. 14, 1 July 2002 (2002-07-01), pages 1121-1135, XP009108470, ISSN: 0141-5492
- PINKSE G G M ET AL: "RGD peptides confer survival to hepatocytes via the beta1-integrin-ILK-pAkt pathway", JOURNAL OF HEPATOLOGY, ELSEVIER, AMSTERDAM, NL, vol. 42, no. 1, 1 January 2005 (2005-01-01), pages 87-93, XP027781301, ISSN: 0168-8278 [retrieved on 2005-01-01]
- HILL, E. ET AL.: 'Designing Scaffolds to Enhance Transplanted Myoblast Survival and Migration' TISSUE ENGINEERING vol. 12, no. 5, 2006, pages 1295 - 1304, XP002471951
- LESLIE-BARBICK, J. E. ET AL.: 'Micron-Scale Spatially Patterned, Covalently Immobilized Vascular Endothelial Growth Factor on Hydrogels Accelerates Endothelial Tubulogenesis and Increases Cellular Angiogenic Responses' TISSUE ENGINEERING: PART A vol. 17, no. 1-2, 2011, pages 221 - 229, XP055246029
- HATAKEYAMA, H. ET AL.: 'Patterned biofunctional designs of thermoresponsive surfaces for spatiotemporally controlled cell adhesion, growth, and thermally induced detachment' BIOMATERIALS vol. 28, no. 25, 2007, pages 3632 - 3643, XP022104742
- Yaowen Liu ET AL: "Hepatocyte Cocultures with Endothelial Cells and Fibroblasts on Micropatterned Fibrous Mats to Promote Liver-Specific Functions and Capillary Formation Capabilities", Biomacromolecules, vol. 15, no. 3, 10 March 2014 (2014-03-10) , pages 1044-1054, XP055362398, US ISSN: 1525-7797, DOI: 10.1021/bm401926k

### SEQUENCE LISTING

<110> Nitto Denko Corporation
<120> Agent for promoting tissue regeneration
<130> PCT2729ND
<160> 11
<170> PatentIn version 3.5
<210> 1
   <211> 4
   <212> PRT
   <213> Artificial sequence
<220>
   <223> RGD peptide
<400> 1
   Arg Gly Asp Ser
   1
<210> 2
   <211> 5
   <212> **PRT**
   <213> **Artificial** sequence
<220>
   <223> RGD peptide
<400> 2
   Gly Arg Gly Asp Ser
   1               5
<210> 3
   <211> 4
   <212> PRT
   <213> Artificial sequence
<220>
   <223> RGD peptide
<400> 3
   Ala Arg Gly Asp
   1
<210> 4
   <211> 6
   <212> PRT
   <213> Artificial sequence
<220>
   <223> RGD pepdide
<400> 4
   Gly Arg Gly Asp Thr Pro
   1               5
<210> 5
   <211> 6
   <212> PRT
   <213> Artificial sequence
<220>
   <223> RGD pepdide
<400> 5
   Gly Arg Gly Asp Ser Pro
   1               5
<210> 6
   <211> 7
   <212> PRT
   <213> Artificial sequence
<220>
   <223> RGD peptide
<400> 6
   Gly Arg Gly Asp Ser Pro Lys
   1               5
<210> 7
   <211> 5
   <212> PRT
   <213> Artificial sequence
<220>
   <223> RGD peptide
<400> 7
   Gly Arg Gly Asp Ser
   1               5
<210> 8
   <211> 11
   <212> PRT
   <213> Artificial sequence
<220>
   <223> RGD peptide
<400> 8
   Ala Cys Asp Cys Arg Gly Asp Cys Phe Cys Gly
   1               5                   10
<210> 9
   <211> 13
   <212> PRT
   <213> Artificial sequence
<220>
   <223> RGD peptide
<400> 9
   Asp Gly Ala Arg Tyr Cys Arg Gly Asp Cys Phe Asp Gly
   1               5                   10
<210> 10
   <211> 5
   <212> PRT
   <213> Artificial sequence
<220>
   <223> RGD peptide
<400> 10
   Cys Arg Gly Asp Cys
   1               5
<210> 11
   <211> 9
   <212> PRT
   <213> Artificial sequence
<220>
   <223> RGD peptide
<400> 11
   Cys Tyr Gly Gly Arg Gly Asp Thr Pro
   1               5

## Description

### [Technical Field]

The present invention relates to a method for promoting cell growth using an RGD peptide, and a method for producing a cell culture using an RGD peptide.

### [Background Art]

When a tissue of living organisms is damaged, it regenerates so as to compensate for the damaged portion, and attempts to restore its function. For example, it is known that when more than half of the liver is removed, the liver can regenerate into almost its original size and restore its function within a short period of time. Studies on tissue regeneration have long been conducted centering on the liver, and various findings have been reported; however, detailed mechanism of tissue regeneration has not yet been clarified. Multiple types of cells are considered to be involved in a complex manner in tissue regeneration; however, even for which one of these cells play a major role, no definitive conclusion has been obtained.

For regeneration of tissues, growth of cells constituting the tissues and of stem cells that differentiate into said cells is essential, and cell growth factors such as HGF are known to be involved in the tissue regeneration via their cell-growth ability (Non-Patent Document 1). However, due to the growing need for recent regenerative medicine, exploration of additional substances that contribute to the promotion of tissue regeneration and cell proliferation has been demanded.

### [Citation List]

Non-Patent Document 1: Nakamura and Mizuno, Proc Jpn Acad Ser B Phys Biol Sci. 2010; 86 (6) : 588-610.

Kishida et al. (Biomaterials 1992;13(13):924-30) describe an RGD-albumin conjugate. Ito et al. (Biomaterials 2005 Nov;26(31):6185-93) report on the effect of RGD peptide-conjugated magnetite cationic liposomes on cell growth and cell sheet harvesting. Park and Bae (Biosci Biotechnol Biochem 2002 Jul;66(7):1473-8) report on the phenotype of hepatocyte spheroids in Arg-Gly-Asp (RGD) containing a thermo-reversible extracellular matrix. Ortega-Velázquez et al. (FASEB J 2003 Aug;17(11):1529-31) report that Arg-Gly-Asp-Ser (RGDS) peptide stimulates Transforming Growth Factor beta1 transcription and secretion through integrin activation. Kiely et al. (Gastroenterology, vol. 124, no. 4, April 2003, page A597) report that RGD peptides enhance cell proliferation during intestinal adaptation. Park (Biotechnology Letters volume 24, 1131-1135 (2002)) reports on the improved long-term culture of hepatocytes in a hydrogel containing Arg-Gly-Asp (RGD). Pinske et al. (J Hepatol 2005 Jan;42(1):87-93) report that RGD peptides confer survival to hepatocytes via the beta1-integrin-ILK-pAkt pathway.

### [Disclosure of Invention]

### Problems to Be Solved by the Invention

The present invention aims to provide a novel method for promoting cell growth

### Means for Solving the Problems

The present inventors have found that, in the course of extensive studies to solve the above problems, RGD peptides promote the growth of cells, and completed the present invention.

Namely, the present invention is as defined by the appended claims.

### Advantageous Effects of Invention

The present invention enables to promote cell growth, and therefore significant contribution in the fields of biology, cell engineering, medicine, and medical care (in particular regenerative medicine) can be expected.

### [Brief Description of Drawings]

[Fig. 1] Figure 1 is a microscopy image of BrdU-positive cells when hepatocytes are cultured in the presence of RGD peptides.
[Fig. 2] Figure 2 is a graph showing the percentage of BrdU-positive cells when hepatocytes are cultured in the presence of RGD peptides.

### [Description of Embodiments]

Unless otherwise defined herein, all technical and scientific terms used herein have the same meaning as those skilled in the art commonly understand.

The present invention is as defined by the appended claims.

In the present specification, the RGD peptide means a peptide comprising RGD motif, i.e., an amino acid sequence consisting of Arg-Gly-Asp. RGD peptides may consist of only Arg-Gly-Asp, or may comprise additional amino acid residues at N-terminal, C-terminal or both terminals. RGD peptides may be, but are not limited to, for example, 3-100 amino acids in length, 3-50 amino acids in length, 3-25 amino acids in length, 3-20 amino acids in length, 3-15 amino acids in length, 3-10 amino acids in length, 3-8 amino acids in length, or 3-5 amino acids in length, etc. Therefore, RGD peptides may be, but are not limited to, for example, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids in length, etc.

RGD peptides may be linear, cyclic or branched. When the RGD peptide is cyclic, it may be in a form wherein both ends of the peptide are bound (head-to-tail type), or in a loop form wherein at least one end may be free. In the case of looped peptide, RGD motif may be located at an apex portion of the loop. Amino acid residues may not be modified, or may be modified. In the case of RGD peptides with 6 amino acids or more in length, they may comprise one or more RGD motifs. RGD peptides can assume a topology in which, at the latest by they contact with cells, at least one RGD motif is recognized by the cells. For example, RGD peptides may assume a topology in which at least one RGD motif is recognized in vitro by cells, or, they may be designed such that while they assume a topology in which RGD motif is not recognized in vitro by cells, they assume a topology in which at least one RGD motif is recognized in vivo by cells due to changes in environment such as pH or due to metabolism. Here, examples of the topology recognized by cells include, but are not limited to, a topology in which RGD motif is not hidden by other amino acid residues when the RGD peptide takes a three-dimensional structure. RGD peptides may have at least one RGD motif constituted by unmodified amino acid residues.

As the RGD peptide, those already known may be utilized, or it may be newly designed. Non-limiting examples of known RGD peptides include Arg-Gly-Asp (e.g., Sigma-Aldrich, Cat. No. A8052) , Arg-Gly-Asp-Ser (SEQ ID NO: 1, for example, Sigma-Aldrich, Cat. No. A9041; Peptide Institute, Cat. No. 4171), Gly-Arg-Gly-Asp-Ser (SEQ ID NO: 2, for example, Sigma-Aldrich, Cat. No. G4391), Cyclo (Ala-Arg-Gly-Asp-3-Aminomethylbenzoyl) (SEQ ID NO: 3, for example, Sigma-Aldrich, Cat. No.C9357), Gly-Arg-Gly-Asp-Thr-Pro (SEQ ID NO: 4, for example, Sigma-Aldrich, Cat. No. G5646), Gly-Arg-Gly-Asp-Ser-Pro (SEQ ID NO: 5, for example, Sigma-Aldrich, Cat. No.SCP0157), Gly-Arg-Gly-Asp-Ser-Pro-Lys (SEQ ID NO: 6, for example, Sigma-Aldrich, Cat. No.G1269), Echistatin (for example, Sigma-Aldrich, Cat. No.SCP0157), GRGDS (SEQ ID NO: 7, for example, Peptide Institute, Cat. No.4189-v), Cilengitide, RGD4C (ACDCRGDCFCG, SEQ ID NO: 8), RGD10 (DGARYCRGDCFDG, SEQ ID NO: 9), Cyclo (Cys-Arg-Gly-Asp-Cys) (SEQ ID NO: 10), SKF 107260, G-4120, CYGGRGDTP (SEQ ID NO: 11), Cyclo (RGDfK), and the like.

In addition, RGD peptides include RGD peptidomimetic that mimics the function of RGD peptides (e.g. , Duggan et al., J Med Chem. 2000; 43(20): 3736-45), and a peptide comprising RLD (Arg-Leu-Asp) motif or KGD (Lys-Gly-Asp) motif having a function similar to the RGD peptides. The function of the RGD peptides includes cell growth-promoting effects as described in Example 1 herein, and binding ability to integrin molecules (e.g., integrins αvβ1, αvβ3, etc.) and the like.

The tissues, the regeneration of which is to be promoted by the composition of the present disclosure, are not particularly limited, and include various tissues throughout the body. Examples of such tissues include, but are not limited to, tissues wherein stellate cells are present, tissues wherein fibrosis occurs, and tissues wherein stem cells are present, and the like. Non-limiting examples of tissues, the regeneration of which is to be promoted, include liver, pancreas, kidney, intestine, lung, spleen, heart, bone marrow, vocal cord, skin, peritoneum, eye, vessel, nervous tissue, cartilage, bone, muscle, breast, and hair follicle, etc.

The composition of the present disclosure is useful for tissue regeneration that occurs in damaged tissues or implanted tissues. Damaged tissues include tissue destruction, inflammation, necrosis, fibrosis, surgical invasion, and the tissues that received organ failure, etc.

Tissue regeneration by the composition of the present disclosure may involve differentiation and/or proliferation of stem cells. Also, tissue regeneration by the composition of the present disclosure may involve growth of tissue parenchyma cells. Differentiation of stem cells can be evaluated by, for example, detection of cellular markers specific to differentiated cells, and detection of functions exhibited by differentiated cells. Cell growth can be evaluated by various known techniques, for example, counting the number of viable cells over time, measurement of size, volume or weight of tissues, measurement of the amount of DNA synthesis, detection of cell growth markers, WST-1 method, BrdU (bromodeoxyuridine) method, and ³H-thymidine incorporation method.

The composition of the present disclosure can be used for a subject having a condition in which tissue regeneration is suppressed. The condition in which tissue regeneration is suppressed includes, without limitation, for example, inflammation, necrosis, fibrosis, organ failure, decreased platelet count, genetic abnormalities, decreased noradrenaline, and the like.

The amount of an RGD peptide blended in the composition of the present disclosure may be an amount that promotes tissue regeneration when the composition is administered. In addition, an amount that does not cause adverse effects exceeding the benefits of administration is preferred. Such an amount is known, or can be appropriately determined by in vitro test using cultured cells, etc., and a test using a model animal such as mouse, rat, dog or pig, and such test methods are well known to those skilled in the art. Promotion of the tissue regeneration can be evaluated by the recovery of function, weight and size of the tissue using biochemical tests and image diagnosis using X-ray, ultrasound, MRI, CT, and an endoscope or the like. The amount of an RGD peptide to be blended may vary depending on the dosage form of the composition. For example, when using multiple units of the composition in a single administration, the amount of an RGD peptide blended in one unit of the composition can be what the amount of the RGD peptide required for one administration is divided by more than one. Adjustment of such amounts can be appropriately carried out by those skilled in the art.

The present disclosure also relates to a method for promoting tissue regeneration in a subject, comprising a step of administering an RGD peptide to a subject in need thereof. The subject of the present method may have disordered tissues, or may have tissue transplant. Disorders include, but are not limited to, tissue destruction, inflammation, necrosis, fibrosis, surgical invasion, and organ failure, etc. In addition, the subject of the present method may be afflicted with a disease that can be improved by tissue regeneration. Such diseases will be described in detail below.

The present disclosure also relates to a composition for promoting cell growth comprising an RGD peptide as an active ingredient, a method for producing a composition for promoting cell growth comprising an RGD peptide as an active ingredient, a use of an RGD peptide for producing a composition for promoting cell growth, a composition comprising an RGD peptide in an amount effective for promoting cell growth, and to an RGD peptide for use in the promotion of cell growth.

The cells, the growth of which is to be promoted include, but are not limited to, for example, cells of tissues such as liver, pancreas, kidney, intestine, lung, spleen, heart, bone marrow, vocal cord, skin, peritoneum, eye, vessel, nerve tissue, cartilage, bone, muscle, breast, and hair follicle. Cells may be somatic cells (i.e., differentiated cells), and stem cells. Somatic cells include parenchymal cells and non-parenchymal cells. Stem cells are not particularly limited, and include, for example, tissue stem cells (somatic stem cells, adult stem cells), embryonic stem cells, iPS cells, etc. Stem cells may be totipotent, pluripotent, multipotent or unipotent. Tissue stem cells include, but are not limited to, neural stem cells, hematopoietic stem cells, mesenchymal stem cells, liver stem cells, pancreatic stem cells, skin stem cells, muscle stem cells, germ stem cells, and cardiac stem cells, etc. The cells may be of autologous, may be of other individuals of the same species or individuals of different species. Cells that have been grown can be transplanted to a subject in need thereof. Furthermore, the cells, the growth of which is to be promoted, may be present in the body, or present in vitro. When present in vitro, the cells can be present in the tissue isolated from a living body, or may be present in a state isolated from the tissue.

The cells may have a cell surface molecule that recognizes RGD motif. Such a cell surface molecule includes, for example, integrin. Integrin is a protein consisting of two subunits, i.e., α chain and β chain. In one embodiment, the cells express integrin with β1 chain. In one embodiment, the cells express integrin with αv chain. In a particular embodiment, the cells express integrin which is selected from the group consisting of integrins α5β1, α8β1, αIIBβ3, αvβ1, αvβ3, αvβ5, αvβ6 and αvβ8.

Cell growth can be evaluated by various known techniques, for example, counting the number of viable cells over time, measurement of size, volume or weight of tissues, measurement of the amount of DNA synthesis, detection of cell growth markers, WST-1 method, BrdU (bromodeoxyuridine) method, and ³H-thymidine incorporation method. In the present invention, promoting cell growth means that, the level of cell growth in the presence of RGD peptides (for example, the number of cells after culturing for a predetermined period, or the rate of increase from the number of cells at a certain point (for example, the number of cells upon inoculation)) is increased compared to the level of cell growth in the absence of RGD peptides. A degree of increases in the cell growth is not limited, and may be, for example, 5% or more, 10% or more, 15% or more, 20% or more, 25% or more, 30% or more, 40% or more, 50% or more, 60% or more, 70% or more, 80% or more, 90% or more, 100% or more, 150% or more, 200% or more, 250% or more, 300% or more, etc. relative to the cell growth in the absence of RGD peptides.

The blended amount or effective amount of an RGD peptide in the composition of the present disclosure may be an amount that promotes cell growth when the composition is administered. Also, an amount that does not cause adverse effects exceeding the benefit of administration is preferable. Such an amount is known, or can be appropriately determined by in vitro test using cultured cells, etc., or a test using a model animal such as mouse, rat, dog or pig, and such test methods are well known to those skilled in the art. Promotion of cell growth can be evaluated by various methods described above. The amount of an RGD peptide to be blended may vary depending on the dosage form of the composition. For example, when using multiple units of the composition in a single administration, the amount of an RGD peptide blended in one unit of the composition can be what the amount of the RGD peptide required for one administration is divided by more than one. Adjustment of such amounts can be appropriately carried out by those skilled in the art. The composition
can be used in vitro, in vivo or ex vivo. The composition can also be used in the method for producing a cell culture of the present invention described later.

The present disclosure also relates to a method for promoting cell growth in a subject, comprising a step of administering an effective amount of an RGD peptide to a subject in need thereof. The subject of the present method may have disordered tissues, or may have tissue transplant. Disorders include, but are not limited to, for example, tissue destruction, inflammation, necrosis, fibrosis, surgical invasion, and organ failure, etc. In addition, the subject of the present method may be afflicted with a disease that can be improved by cell growth. Such diseases will be described in detail below.

The present disclosure also relates to a composition for treating diseases which can be ameliorated by tissue regeneration and/or cell growth, comprising an RGD peptide as an active ingredient, a method for producing a composition for treating said diseases comprising blending an RGD peptide as an active ingredient, a use of an RGD peptide in producing a composition for treating said diseases, an RGD peptide for use in the treatment of said diseases, a composition comprising an effective amount of an RGD peptide for treating said diseases, and a method for treating said diseases comprising administering an effective amount of an RGD peptide to a subject in need thereof.

Diseases which can be improved by tissue regeneration and/or cell growth include, but are not limited to, diseases accompanied by disappearance of cells or cellular substances in tissues, and diseases requiring transplantation of cells or tissues, and the like. Disappearance of cells and cell substrates can be caused by, without limitation, for example, cell death due to necrosis or apoptosis or the like, tissue damage due to external wound, removal of tissues by surgery, and the like. Diseases which can be ameliorated by tissue regeneration and/or cell growth may develop in the tissues such as liver, pancreas, kidney, intestine, lung, spleen, heart, bone marrow, vocal cord, skin, peritoneum, eye, vessel, nerve tissue, cartilage, bone, muscle, breast, hair follicle and the like. Non-limiting examples of diseases that may be ameliorated by tissue regeneration and/or cell growth include, for example, tissue fibrosis (e.g., liver fibrosis (including cirrhosis of the liver), pulmonary fibrosis, renal fibrosis, pancreatic fibrosis, intestinal fibrosis, myelofibrosis, peritoneal fibrosis, etc.), infarct diseases (e.g., myocardial infarction, cerebral infarction, etc.), necrotizing diseases (e.g., gangrene (including diabetic gangrene), pressure ulcer, ulcer etc.), degenerative nerve diseases, external wound, organ dysfunction requiring transplantation of cells and tissues, and tumor diseases requiring reconstruction (e.g., breast cancer, head and neck cancer, tongue cancer, oral cavity cancer, esophageal cancer, etc.).

Various compositions of the present disclosure may further comprise, in addition to RGD peptides, a cell growth factor as an active ingredient, or they may be used in combination with a cell growth factor. Likewise, in various methods of the present invention, RGD peptides can be used in combination with a cell growth factor. Cell growth factors include, but are not limited to, HGF, EGF, VEGF, FGF, TGF-α, PDGF, HB-EGF, neurotrophin, GDNF, etc. The cell growth factor may be either naturally occurring or artificially produced. Thus, the cell growth factor includes a recombinant cell growth factor. Information on the amino acid of cell growth factors and the base sequence encoding it can be obtained from existing databases (e.g., GenBank etc.).

The cell growth factor in the present invention includes functional variants thereof. When the cell growth factor is protein, its functional variants include, but are not limited to, the following: (i) a variant having one or more, typically one or several mutations in the amino acid sequence of said protein, and still having equivalent functions of said protein, (ii) a variant encoded by a nucleic acid having one or more, typically one or several mutations in the base sequence of the nucleic acid that has the base sequence of the gene encoding said protein, or that encodes the same polypeptide as this nucleic acid, and having equivalent functions, (iii) a variant encoded by a nucleic acid that hybridizes the complementary strand, or a fragment thereof, of the nucleic acid that has the base sequence of the gene encoding said protein, or that encodes the same polypeptide as this nucleic acid, or that encodes the variant of (ii), under stringent conditions, and having equivalent functions of said protein, (iv) a variant having an amino acid sequence that has 60% or more, preferably 70% or more, more preferably 80% or more, still more preferably 90% or more, and particularly preferably 95% or more homology to the amino acid sequence of said protein, and having equivalent functions of said protein, (v) a variant encoded by a nucleic acid that has 60% or more, preferably 70% or more, more preferably 80% or more, still more preferably 90% or more, and particularly preferably 95% or more homology to the base sequence of the gene encoding said protein, and having equivalent functions of said protein, and the like.

Those skilled in the art can appropriately produce the above functional variants based on the sequence information of the cell growth factor, using any known techniques, such as chemical synthesis, cleavage or insertion of nucleic acid by restriction enzyme, site-specific mutagenesis, irradiation or ultraviolet irradiation.

Whether or not a certain variant has equivalent functions as those of the cell growth factor can be evaluated by analyzing said variant in terms of known functions of the cell growth factor, for example, without limitation, an ability to induce cell proliferation, using any known method, and by comparing it with an appropriate negative control and the cell growth factor as a positive control. For example, in one variant, if the above function is better than the negative control, for example, 10% or more, 25% or more, 50% or more, 75% or more, and even 100% or more better than that of the negative control, and/or, if this function is 1/100 or more, 1/50 or more, 1/25 or more, 1/10 or more, 1/5 or more, and even 1/2 or more of that of the positive control, then this variant is included in the functional variant of the cell growth factor.

The term "stringent conditions" as used herein refers to well-known parameters in the art, which are described in standard protocols such as Sambrook et al. , Molecular Cloning: A Laboratory Manual, 3d ed., Cold Spring Harbor Press (2001) ; Ausubel et al. , Current Protocols in Molecular Biology, Greene Publishing Associates (1992); and others.

Stringent conditions in the present invention refer to, for example, hybridization at 65°C using a hybridization buffer consisting of 3.5 x SSC (0.15 M sodium chloride/0.15 M sodium citrate, pH 7), 0. 02% Ficoll, 0.02% polyvinyl pyrrolidone, 0.02% bovine serum albumin, 25 mM NaH₂PO₄ (pH 7), 0.05% SDS, and 2 mM EDTA. After hybridization, the membrane to which DNA has been transferred is washed with 2 x SSC at room temperature, then with 0.1-0.5 x SSC/0.1 x SDS up to the temperature of 68°C. Alternatively, stringent hybridization may be performed using a commercially available hybridization buffer, such as ExpressHyb(R) Hybridization Solution (Clontech Laboratories, Inc.) under hybridization and washing conditions described by the manufacturer.

Other available conditions and reagents which result in comparable stringency exist; because those skilled in the art are considered to be familiar with such conditions, these are not specifically described herein. However, it is possible to manipulate the conditions in order to enable clear identification of nucleic acids encoding protein variants.

The cell growth factor and/or a functional variant thereof of the present invention include, in addition to said protein itself or a functional variant thereof, a nucleic acid molecule encoding said protein or a functional variant thereof.

When active ingredients of various compositions disclosed herein and methods of the invention described herein comprise a nucleic acid (for example, a nucleic acid molecule encoding a cell growth factor), these may be used as a bare nucleic acid as it is, or these may be supported by various vectors. As the vector, any publicly known vectors such as plasmid vectors, phage vectors, phagemid vectors, cosmid vectors, and viral vectors can be used. Preferably, the vector comprises at least a promoter that enhances the expression of a supporting nucleic acid; in this case, the nucleic acid is preferably operably linked to such promoter. The phrase "nucleic acid is operably linked to promoter" means that the nucleic acid and the promoter are located such that the protein encoded by the nucleic acid can be suitably produced by the action of the promoter. The vector may or may not be capable of replication in a host cell, and transcription of genes may take place outside the nucleus of the host cell, or may take place in the nucleus. In the latter case, the nucleic acid may be incorporated into the genome of the host cell.

Furthermore, active ingredients can be supported on a variety of non-viral lipids or protein carriers . Examples of such carriers include, without limitation, cholesterol, liposomes, antibody protomers, cyclodextrin nanoparticles, fusion peptides, aptamers, biodegradable polylactic acid copolymers, polymers and the like, and they can enhance the incorporation efficiency into cells (for example, see Pirollo and Chang, Cancer Res. 2008; 68 (5): 1247-50, etc.). In particular, cationic liposomes and polymers (such as polyethyleneimine) are useful. Further examples of useful polymers as such carriers include those described in US 2008/0207553, US 2008/0312174, etc.

Various compositions as described herein can be used in medical applications such as in vivo tissue regeneration and treatment of diseases. Thus, various compositions of the present disclosure can be pharmaceutical compositions. In a pharmaceutical composition, as long as effectiveness of active ingredients is not interfered, the active ingredients may be combined with other optional ingredients. Such optional ingredients include, for example, other chemotherapeutic agents, pharmaceutically acceptable carriers, excipients, and diluents, etc. Also, depending on the administration route and the drug release manner, the composition may be coated with a suitable material, for example, enteric coating and timed-disintegrating material, and the composition may be incorporated into appropriate drug release systems.

Various compositions as described herein (including various pharmaceutical compositions) may be administered via various routes including both oral and parenteral routes, for example, without limitation, oral, intravenous, intramuscular, subcutaneous, topical, intratumoral, rectal, intraarterial, intraportal, intraventricular, transmucosal, transdermal, intranasal, intraperitoneal, intrapulmonary, and intrauterine routes, and they may be formulated in a dosage form suitable for each administration route. Such dosage forms and formulation methods may be selected as appropriate from any known ones (see, for example, "Hyojun Yakuzaigaku" (Standard Pharmaceutical Science), Ed. by Yoshiteru Watanabe, et al., Nankodo, 2003).

Examples of dosage forms suitable for oral administration include, but are not limited to, powders, granules, tablets, capsules, solutions, suspensions, emulsions, gels, and syrups, and examples of dosage forms suitable for parenteral administration include injections such as an injectable solution, an injectable suspension, an injectable emulsion, and an injection in a form that is prepared at the time of use. Formulations for parenteral administration may be in the form of transplantation, or aqueous or nonaqueous isotonic sterile solutions or suspensions.

Various compositions as described herein (including various pharmaceutical compositions) may be supplied in any forms; however, from the viewpoint of storage stability, they may be supplied in a form that can be prepared at the time of use, for example, in a form that can be prepared at the site of clinical practice or in the vicinity thereof by a physician and/or pharmacist, nurse or other paramedical personnel, etc. Such a form is particularly useful when the composition comprises a component that is difficult to store in a stable manner, such as lipids, proteins, and nucleic acids. In this case, the composition
is provided in one or more containers which further comprise at least one of essential constituents, and the composition is prepared, for example, within 24 h, preferably 3 h prior to use, and more preferably immediately before use. Upon preparation, reagents, solvents and dispensing equipment usually available in a place of preparation can be used as appropriate.

Accordingly, the present disclosure also relates to a kit or pack for preparation of a composition, comprising one or more containers containing singly or in combination active ingredients which may be included in various compositions of the present disclosure and also relates to a constituent element necessary for various compositions provided in the form of such a kit or pack. The kit or pack may further comprise, in addition to the above, instructions describing a method of preparation and a method of use (such as administration method) of the various compositions of the present disclosure , for example, an instruction, or a medium wherein the instruction is recorded, for example electronic recording media such as flexible disc, CD, DVD, blue-ray disc, memory card and USB memory, etc. Furthermore, the kit or pack may include all of the constituent elements for completing the various compositions, but need not necessarily include all of the constituent elements. Therefore, the kit or pack may not include a reagent or a solvent usually available at the site of clinical practice and experimental facility, such as sterile water, physiological saline, and glucose solution, etc. The kit or pack may be used for the above-mentioned variety of applications regarding the composition of the present disclosure such as promotion of cell growth, promotion of tissue regeneration, and production of cell culture and the like.

An effective amount in the various methods of the invention described herein may be, for example, with respect to regeneration of tissues, an amount that promotes tissue regeneration, or eliminates delay in tissue regeneration; with respect to growth of cells, an amount that promotes cell growth, or eliminates delay in cell growth; with respect to treatment of a disease, an amount that reduces symptoms of the disease, or delays or stops progression of the disease, and preferably, an amount that suppresses or cures the disease. In addition, an amount that does not cause an adverse effect exceeding the benefits from administration is preferred. Such an amount can be appropriately determined by an in vitro test using cultured cells, or a test in a model animal such as mouse, rat, dog or pig, and such test methods are well known to those skilled in the art. Furthermore, dosage of a drug used in the treatment methods of the disclosure is either known to those skilled in the art or can be appropriately determined by the above-mentioned tests, etc. A total amount of administration per day of an RGD peptide may be, without limitation, for example approximately 1 µg/kg to approximately 1000 mg/kg of body weight, approximately 10 µg/kg to approximately 100 mg/kg of body weight, or approximately 100 µg/kg to approximately 10 mg/kg of body weight, etc.

Specific dosage of an active ingredient to be administered in the treatment methods of the disclosure described herein can be determined in consideration of various conditions related to a subject requiring treatment, for example, presence or absence of conditions which inhibit tissue regeneration or cell growth, severity of symptoms, general health, age, body weight of the subject, gender of the subject, diet, time and frequency of administration, pharmaceutical agents that are used in combination, responsiveness to therapy, dosage form, and compliance to therapy.

The administration route includes various routes including both oral and parenteral routes, for example, oral, intravenous, intramuscular, subcutaneous, topical, intratumoral, rectal, intraarterial, intraportal, intraventricular, transmucosal, transdermal, intranasal, intraperitoneal, intrapulmonary, and intrauterine routes.

The frequency of administration differs depending on the properties of a composition used and conditions of a subject including those described above; and the frequency may be, for example, multiple number of times per day (i.e., 2, 3, 4, or 5 times or more per day) , once a day, every several days (i.e. , every 2, 3, 4, 5, 6, or 7 days, etc.), every week, or every few weeks (i.e., every 2, 3, or 4 weeks, etc.).

As used herein, the term "subject" means any living individual, preferably an animal, more preferably a mammal, more preferably a human individual. The subject may be healthy, or may be suffering from some disease; in the case where treatment of a particular disease is contemplated, typically the subject means a subject suffering from such disease, or a subject having a risk of suffering from such disease.

In addition, the term "treatment" includes, as used herein, medically acceptable all kinds of preventive and/or therapeutic intervention for the purpose of cure, temporary remission or prevention of disease. For example, the term "treatment" includes medically acceptable intervention with variety of purposes, including delay or stop of progression of disease, regression or disappearance of lesions, prevention of onset or prevention of recurrence.

The present invention also relates to a method for promoting cell growth comprising a step of contacting cells with an RGD as defined by the appended claims peptides, as defined by the appended claims. This method is performed in vitro, The step of contacting cells with an RGD peptide can be carried out in the presence of serum and/or a cell growth factor. RGD peptides, cells, promotion of cell growth and cell growth factors are as described above for the composition for promoting cell growth.

The serum includes xenogeneic serum and allogeneic serum. Xenogeneic serum means serum derived from an organism of a species different from that of the cell to be contacted with the RGD peptide. For example, when the cell is a human cell, serum derived from cattle or horse, such as fetal bovine serum (FBS, FCS), calf serum (CS), horse serum (HS) and the like correspond to xenogeneic serum. "Allogeneic serum" means serum derived from an organism of the same species as the cell. For example, when the cell is a human cell, human serum corresponds to allogeneic serum. Allogeneic serum includes autologous serum, i.e. , the serum of the individual from which the cell is derived, as well as allogeneic foreign serum derived from an allogeneic individual other than said individual. In this specification, serum other than autologous serum, that is, xenogeneic serum and allogeneic foreign serum may be collectively referred to as non-autologous serum in some cases. The concentration of the serum may be in the range of, for example, without limitation, 0.1-50 v/v%, 0.5-40 v/v%, 1-30 v/v%, and 2-20 v/v%, etc.

The contact between an RGD peptide and cells is not particularly limited as long as it is in such a manner that the RGD peptide can promote the growth of the cells. For example, the RGD peptide may be added to a medium containing the cells (for example, a medium, and physiological buffer, etc.) or the RGD peptide may be administered to a subject or tissue containing the cells. The concentration of the RGD peptide upon contacting with the cells is not limited, and may be in the range of, for example, 0.1 µg/mL to 100 mg/mL, 1 µg/mL to 10 mg/mL, 10 µg/mL to 1 mg/mL, 20 µg/mL to 500 µg/mL, 25 µg/mL to 400 µg/mL, 50 µg/mL to 100 µg/mL, and the like.

The present invention also relates to a method for producing a cell culture, comprising a step of contacting cells with an RGD as defined by the appended claims peptide, and a step of culturing the cells, as defined by the appended claims. The step of contacting cells with an RGD peptide and/or the step of culturing the cells can be carried out in the presence of serum and/or a cell growth factor. RGD peptides, cells, promotion of cell growth, a step of contacting cells with an RGD peptide, serum, and cell growth factors, etc. are as described above for a composition for promoting cell growth or a method for promoting cell growth.

Culture of cells in the present invention can be carried out under conditions suitable for survival and growth of the cells. Such conditions are known to those skilled in the art, or can be appropriately adjusted by those skilled in the art. Non-limiting examples of such conditions include culturing in a cell culture medium at 37°C and 5% CO₂, and the like. Examples of the cell culture medium include, but are not limited to, DMEM, MEM, F12, DME, RPMI1640, MCDB (MCDB 102, 104, 107, 131, 153, 199, etc.), L15, SkBM, RITC80-7, DMEM/F12 and the like. The medium may or may not comprise serum.

A cell culture produced by the production method of the present invention can be used for various transplantation therapies and the like. Therefore, the cell culture produced by the production method of the present invention may be those used for transplantation therapy. Since the production method of the present invention enables rapid production of a cell culture having a desired number of cells, it is useful for the production of cell cultures in transplantation therapy and the like.

### [Example]

The present invention will be explained in more detail in the following example, which is merely illustrative and does not limit the present invention in any way.

### Example 1. Effects of RGD peptide on hepatocyte growth

Rat liver parenchymal cells collected from the liver of a GFP gene-introduced rat (Slc Japan) by perfusion of collagenase solution from the portal vein (Seglen, Methods Cell. Biol. (1976) 13: 29-83) were inoculated to a 60-mm dish coated with rat tail-derived Native collagen I (Sigma-Aldrich) with a concentration of 10 µg/cm², at a concentration of 2×10⁵ cells/ml in a DME/F12 medium containing 10% fetal bovine serum, to which different concentrations of the following peptides were added and cultured at 37°C and 5% CO₂ for 48 h: RGD peptide (Sigma-Aldrich, Cat. No. A8052): 50 µg/ml, 100 µg/ml; GRGES peptide (Peptide Institute, Cat. No. PFA-3907-PI): 100 µg/ml; GRGDS peptide (Peptide Institute, Cat. No. 4189-v): 50 µg/ml, 100 µg/ml. At 48 h of culturing, the medium was replaced with a DME/F12 medium containing 10 µM BrdU and 10% FBS, and it was incubated at 37°C and 5% CO₂ for additional 24 h. At 24 h of culturing, the medium was removed, and the resulting sample was washed with PBS, to which 4% PFA was added and fixed at room temperature for 30 min. After fixation, the sample was washed with PBS, and permeation treatment was carried out by adding PBS containing 2-N HCl and 0.1% Triton X-100.

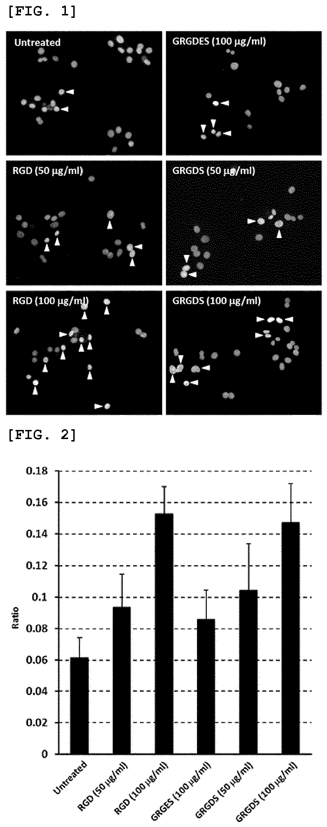

After washing with PBS, blocking was carried out with 5% goat serum, then mouse monoclonal anti-BrdU antibody (MBL) and rabbit polyclonal anti-GFP antibody (Life Technologies) were added, and a primary antibody reaction was carried out at 37°C for 60 min. Following the primary antibody reaction, the sample was washed with PBS, to which Alexa Fluor^{(R)} 488-labeled goat anti-rabbit IgG (Life Technologies) and Alexa Fluor^{(R)} 555-labeled goat anti-mouse IgG (Life Technologies) were added, and a secondary antibody reaction was carried out at 37°C for 60 min. After the secondary antibody reaction, the sample was washed with PBS and cells were encapsulated using an encapsulant containing DAPI. Fluorescence microscopy images are shown in Fig. 1. In the figure, arrow heads indicate BrdU positive cells. In addition, in order to calculate the proportion of BrdU positive cells, the number of BrdU positive cells and the number of GFP positive cells were counted. Figure 2 shows the ratio of BrdU positive cells (grown hepatocytes) to GFP positive cells (entire hepatocytes) .

The results shown in Figs. 1 and 2 indicate that RGD peptides promote the growth of hepatocytes.

## Claims

1. A method for promoting cell growth, comprising a step of contacting cells of liver with the peptide GRGES, wherein the method is performed in vitro, and wherein the peptide is added to the medium containing the cells.

2. The method according to claim 1, wherein the step of contacting cells of liver with the peptide is carried out in the presence of serum and/or a cell growth factor.

3. A method for producing a cell culture, comprising a step of contacting cells of liver with the peptide GRGES, and a step of culturing the cells, wherein the peptide is added to the medium containing the cells.

4. The method according to claim 3, wherein the step of contacting cells of liver with the peptide, and/or the step of culturing the cells of liver is/are carried out in the presence of serum and/or a cell growth factor. 1

## Patentansprüche

1. Verfahren zum Fördern des Zellwachstums, umfassend einen Schritt des Inkontaktbringens von Zellen der Leber mit dem Peptid GRGES, wobei das Verfahren *in vitro* durchgeführt wird und wobei das Peptid zu dem die Zellen enthaltenden Medium zugegeben wird.

2. Verfahren gemäß Anspruch 1, wobei der Schritt des Inkontaktbringens von Zellen der Leber mit dem Peptid in Gegenwart von Serum und/oder eines Zellwachstumsfaktors durchgeführt wird.

3. Verfahren zum Produzieren einer Zellkultur, umfassend einen Schritt des Inkontaktbringens von Zellen der Leber mit dem Peptid GRGES und einen Schritt des Kultivierens der Zellen, wobei das Peptid zu dem die Zellen enthaltenden Medium zugegeben wird.

4. Verfahren gemäß Anspruch 3, wobei der Schritt des Inkontaktbringens von Zellen der Leber mit dem Peptid und/oder der Schritt des Kultivierens von Zellen der Leber in Gegenwart von Serum und/oder eines Zellwachstumsfaktors durchgeführt wird/werden.

## Revendications

1. Procédé permettant de favoriser la croissance cellulaire, comprenant une étape de mise en contact de cellules hépatiques avec le peptide GRGES, dans lequel le procédé est réalisé in vitro, et dans lequel le peptide est ajouté au milieu contenant les cellules.

2. Procédé selon la revendication 1, dans lequel l'étape de mise en contact de cellules hépatiques avec le peptide est réalisée en la présence de sérum et/ou d'un facteur de croissance cellulaire.

3. Procédé permettant de produire une culture cellulaire, comprenant une étape de mise en contact de cellules hépatiques avec le peptide GRGES, et une étape de mise en culture des cellules, dans lequel le peptide est ajouté au milieu contenant les cellules.

4. Procédé selon la revendication 3, dans lequel l'étape de mise en contact des cellules hépatiques avec le peptide, et/ou l'étape de mise en culture des cellules hépatiques est/sont réalisée(s) en la présence de sérum et/ou d'un facteur de croissance cellulaire.
